# DEMANDE DE BREVET EUROPEEN

(11) **EP 0 868 886 A1**
(43) Date de publication de la demande: **07.10.1998**
(21) Numéro de dépôt: 98400772.4
(22) Date de dépôt: 01.04.1998
(51) Int. Cl.: A61B 19/00, A61C 1/08

(54) **Procédé et dispositif de marquage et de repérage d'une zone à traiter dans un organisme**

(30) Priorité: 01.04.1997 FR 9703959
(71) Demandeur: Derycke, Raymond-René, 75116 Paris (FR)
(72) Inventeur: Derycke, Raymond-René, 75116 Paris (FR)
(74) Mandataire: Moncheny, Michel

(57) **Abrégé**

Le procédé de repérage d'une zone à traiter d'un organisme consiste :
- à fixer sur la zone, un ensemble initial de marques (58A,58B) formant des points de référence pouvant apparaître sur des images produites par un dispositif d'imagerie médicale,
- à réaliser des images numérisées prises par rapport à l'ensemble de points de référence (58A,58B),
- à fixer sur ladite zone, un nouvel ensemble de marques optiques (56A,56B), le nouvel ensemble de marques se trouvant dans une position déterminée par rapport à celle de l'ancien ensemble de marques (58A,58B), et
- à repérer le positionnement d'un outil (14), par repérage optique direct de la position de l'outil par rapport audit nouvel ensemble de marques optiques (56A,56B) à l'aide de moyens d'acquisition optiques ((18) placés en vue directe dudit nouvel ensemble de marques (56A,56B), et sensibles à la position de l'outil.

## Description

La présente invention a trait à un procédé et à un dispositif de marquage et de repérage d'une zone à traiter dans un organisme vivant, ce procédé et ce dispositif étant utilisables notamment dans le domaine de la chirurgie et présentant un intérêt particulier dans le domaine de la chirurgie dentaire, crânienne ou généralement orthopédique.

Lors de la réalisation d'opérations chirurgicales, il est fréquent que le chirurgien doive suivre, par un procédé de stéréotaxie, le mouvement de l'outil qu'il manie, sur un écran de visualisation.

A cet effet, il utilise une installation médicale pour l'obtention d'images numérisées de l'organe devant être traité par l'outil. Un dispositif de positionnement de l'outil dans l'espace est en outre mis en oeuvre. Il permet de déterminer en continu la position de l'outil par rapport à l'organe traité. Ce dispositif de positionnement de l'outil est relié à une unité centrale de traitement d'informations comportant des moyens d'affichage d'un représentant graphique de l'outil par rapport à une image numérique de l'organe. Les images numérisées qui sont produites par l'installation d'imagerie médicale ou par rapport à la représentation de celui-ci sont préalablement chargées ou acquises en cours de procédure et mémorisées dans ladite unité centrale de traitement d'informations en vue de leur affichage.

Le dispositif de positionnement de l'outil nécessite la connaissance de points de référence fixes.

Ces points de référence sont généralement pris sur une structure fixe disposée à proximité du patient. Le patient, et en particulier l'organe à traiter, doivent être immobilisés par rapport à cette structure fixe.

En effet, tout déplacement relatif entre l'organe à traiter et les points de référence conduirait à une représentation erronée de la position de l'outil par rapport à l'organe.

Ainsi, les installations de l'état de la technique sont peu fiables et ne sont pas utilisables pour des organes ne pouvant pas être immobilisés par rapport à une structure fixe.

En particulier, dans le cas de la chirurgie dentaire, où il est impossible d'immobiliser une mâchoire du patient lors de l'opération, une telle installation ne peut être mise en oeuvre.

De plus, il est difficile dans les installations actuelles de repérer avec précision, par rapport aux points de référence, la zone de l'organe devant être traitée, et en particulier de déterminer la position exacte des images de la zone à traiter par rapport à cet ensemble de points de référence.

Une solution a été proposée dans le cas de la chirurgie crânienne. Elle consiste à placer à demeure un ensemble de marques pouvant apparaître sur des images produites par le dispositif d'imagerie médicale. Ces marques permettent le positionnement des images numériques prises préalablement par rapport à l'ensemble de points de référence.

Avec cette technique, les marques restent fixées sur le crâne entre le moment où les images numériques sont prises et le moment où l'intervention chirurgicale est pratiquée. Un délai de plusieurs jours peut s'écouler entre ces deux opérations. Aussi, de telles marques ne sont pas adaptées à d'autres secteurs de la chirurgie, par exemple, à la chirurgie dentaire puisqu'il est impossible de laisser celles-ci implantées plusieurs jours dans la bouche du patient avant de pratiquer l'intervention chirurgicale.

L'invention a pour but de proposer un procédé et des dispositifs de marquage et de repérage permettant de visualiser avec précision, par exemple à partir d'une installation de contrôle visuel, un outil par rapport à un organe, par exemple la mâchoire d'un être vivant lors du traitement chirurgical, qui ne présentent pas les inconvénients de l'art antérieur.

Un autre objectif de l'invention est de fournir un tel procédé et dispositif, qui permettent d'obtenir une très grande précision du repérage de l'instrument pendant l'opération.

Un autre objectif encore de l'invention est de fournir un tel procédé et dispositif qui simplifient considérablement les calculs des moyens de traitement numérique utilisés, et qui par conséquent permettent d'obtenir très rapidement l'image précise et repérée de l'instrument par rapport à l'image restituée de la zone.de l'organisme.

L'invention a pour objet un procédé de marquage et de repérage d'une zone à traiter d'un organisme vivant, et notamment d'une zone non ou mal accessible à la vue du médecin ou chirurgien, comprenant les étapes consistant :
- à fixer sur ladite zone, ou sur une autre zone anatomiquement reliée de façon indéformable à ladite zone, un ensemble initial de marques formant des points de référence pouvant apparaître sur des images produites par un dispositif d'imagerie médicale,
- à réaliser une ou des images numérisées prises par rapport à l'ensemble de points de référence,
- à fixer sur ladite zone ou une autre zone, un nouvel ensemble de marques optiques, c'est-à-dire exploitable par une acquisition optique, formant des points de référence, le nouvel ensemble de marques se trouvant dans une position parfaitement déterminée par rapport à celle de l'ancien ensemble de marques, et
- à repérer le positionnement d'un outil ou instrument de traitement au niveau de ladite zone à traiter, par repérage optique direct de la position de l'outil ou instrument par rapport audit nouvel ensemble de marques optiques à l'aide de moyens d'acquisition optiques placés en vue directe dudit nouvel ensemble de marques, et sensibles à la position de l'outil.

Au sens de l'invention, on entend, par position ou positionnement par rapport à un repère formé par un ensemble de marques, l'ensemble des coordonnées spatiales qui définissent, dans l'espace, la position et l'orientation d'un objet.

Avantageusement, après la réalisation de la ou chaque image numérisée, on retire ledit ensemble de points de référence et on fixe ultérieurement ledit nouvel ensemble de marques optiques se trouvant dans une position parfaitement déterminée par rapport à celle de l'ancien ensemble de marques.

Dans un mode de mise en oeuvre particulièrement préféré de l'invention, lesdits moyens d'acquisition optiques sont directement portés par l'outil ou instrument de traitement, de sorte qu'ils peuvent transmettre, à l'extérieur de l'organisme traité, les données permettant de déterminer le positionnement de l'outil par rapport audit nouvel ensemble de marques.

Le nouvel ensemble de marques peut être directement lié géométriquement à la position occupée par l'ancien ensemble de références fixes ou fixées, qui a servi à l'acquisition de l'image numérisée de la zone de l'organisme. Cependant, comme l'on préfère généralement que ce dernier ensemble de marques soit disposé directement sur la zone à traiter, à condition de suivre fidèlement le mouvement de celui-ci, on utilisera généralement un nouvel ensemble de marques optiques mis en place dans des positions distinctes à celles des marques de l'ensemble initial de marques, mais dont les coordonnées par rapport au système de repère ou référence constitué par l'ancien ensemble de marques, sont connues avec une grande exactitude, par exemple grâce à l'utilisation de moyens de fixation susceptibles d'être disposés exactement au même endroit où avaient été disposés les moyens de fixation de l'ensemble initial de références.

L'invention a également pour objet un dispositif de marquage et de repérage destiné à la mise en oeuvre du procédé selon l'invention, caractérisé en ce qu'il comporte un ensemble de marques optiques associé à un support muni de moyens de fixation sur ladite zone ou autre zone anatomiquement reliée de façon indéformable, dans une position de l'ensemble de marques optiques parfaitement déterminée par rapport à la position de l'ensemble initial de marques.

De préférence, ces moyens de fixation sont agencés pour être fixés exactement à l'emplacement sur lequel ont été initialement fixés les moyens de fixation du support de l'ensemble initial de marques destiné à l'acquisition par le dispositif d'imagerie médicale, les positions des marques dudit nouvel ensemble de marques par rapport auxdits moyens de fixation et donc par rapport à l'ensemble initial de marques, étant exactement connues, ce qui permet, sur l'image de l'organe à traiter, d'utiliser le repère de référence formé par le nouvel ensemble de marques pour calculer et représenter la position de l'outil ou instrument par rapport à l'organe à traiter et, s'il y a lieu, à son image.

De façon avantageuse, on peut utiliser dans le dispositif de marquage et de repérage selon l'invention, les mêmes moyens de fixation en position précise que ceux utilisés pour fixer l'ensemble initial de marques, chacun des ensembles de marques étant porté par un support respectif de géométrie parfaitement connue et qui vient se fixer sur lesdits moyens de fixation et de positionnement de façon amovible et précise.

Dans le cas où la zone à traiter est une zone de mâchoire, il est avantageux de réaliser les moyens de fixation en position sous forme d'une empreinte dentaire prise sur la mâchoire et sur laquelle a été fixé, de façon amovible, l'ensemble initial de marques, de sorte qu'en mettant en place l'empreinte exactement dans sa position initiale, le nouvel ensemble de marques fixé sur ladite empreinte occupera une position parfaitement définie, c'est-à-dire connue par rapport à celle de l'ensemble initial de marques qui avait été fixé sur l'empreinte.

Dans le cas où la technique de l'empreinte dentaire, ou d'une empreinte analogue à l'empreinte dentaire, ne peut pas être utilisée, les moyens de fixation en position doivent être agencés pour pouvoir retrouver exactement la même position au niveau ou à proximité de la zone à traiter. Dans le cas de la chirurgie osseuse, ces moyens de fixation peuvent alors, par exemple, coopérer avec des vis ou implants fixes laissés en place dans la zone à traiter.

Les moyens d'acquisition optique susceptibles de déterminer la position de l'outil ou instrument par rapport à l'ensemble de marques optiques sont, selon un perfectionnement particulièrement préféré de l'invention, directement portés par l'outil ou l'instrument, et l'invention a donc également pour objet un outil ou instrument de traitement portant des moyens optiques permettant de repérer la position de l'instrument par rapport aux marques optiques du nouvel ensemble de marques.

De préférence, ces moyens optiques comprennent des fibres optiques se terminant par une ou des optiques appropriées disposées sur ledit outil ou instrument, ces fibres émergeant à distance de l'organisme, loin de l'instrument, pour être raccordées à des moyens permettant, à partir des images optiques des marques transmises par les fibres, de déterminer la position exacte de l'outil ou instrument par rapport aux repères formés par ledit nouvel ensemble de marques.

De façon avantageuse, l'instrument peut également comporter une ou plusieurs sources lumineuses susceptibles d'éclairer lesdites marques optiques. Ces sources lumineuses peuvent être constituées par les extrémités, pourvues d'optiques diffusantes convenables, de fibres optiques aboutissant à l'instrument et reliées, à leurs autres extrémités, à une source lumineuse.

L'invention a également pour objet l'ensemble constitué par un dispositif de marquage et de repérage selon l'invention et un outil ou instrument de traitement portant lesdits moyens d'acquisition optique.

L'invention sera mieux comprise à l'aide de la description qui va suivre, donnée uniquement à titre d'exemple et faite en se référant aux dessins annexés, sur lesquels :
- la Fig.1 est une vue schématique partiellement en perspective avec arrachement partiel d'un dispositif selon l'invention;
- la Fig.2 est une vue en perspective éclatée du dispositif de repérage utilisé dans l'installation selon l'invention; et
- la Fig.3 est une vue éclatée en section du dispositif de la figure 2.

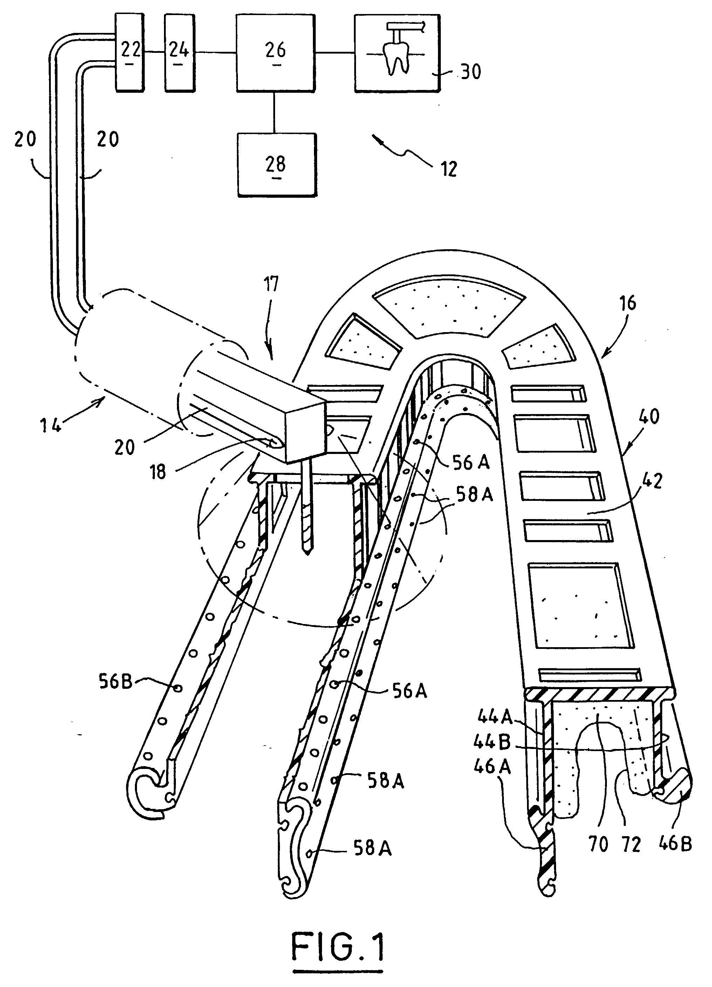

L'installation représentée sur la figure 1, comporte essentiellement une station 12 de visualisation d'un instrument travaillant sur un organisme vivant, un outil où instrument de traitement 14 relié à cette station et un support 16 adapté pour supporter d'une part un premier ensemble de marques formant des points de référence pouvant apparaître sur des images produites par un dispositif d'imagerie médicale et d'autre part, un second ensemble de marques optiques, c'est-à-dire exploitables par une acquisition optique et formant également des points de référence.

L'instrument 14 est formé par exemple par une fraiseuse destinée au traitement des dents. L'installation décrite ici est adaptée à la chirurgie dentaire.

L'installation comporte des moyens optiques 17 de saisie d'une image numérique de la zone traitée. Ils comportent des moyens d'acquisition optiques 18, portés par l'instrument 14, et reliés par des fibres optiques 20 à des moyens 22 de prise d'images eux-mêmes reliés à une unité de traitement d'images 24.

Les moyens d'acquisition optiques sont formés par des optiques diffusantes, par exemple des prismes. Ces moyens sont disposés à l'extrémité de travail de la fraiseuse et sont adaptés pour couvrir la zone de travail.

Dans l'exemple représenté, deux prismes 18 sont disposés de part et d'autre de l'extrémité de travail. Les deux fibres optiques 20 qui relient les prismes 18 aux moyens de prise d'images 22 s'étendent longitudinalement le long du corps de l'instrument 14.

Les moyens de prise d'images 22 sont formés par exemple par deux caméras numériques. L'unité de traitement d'images 24 comporte des moyens d'analyse d'images numérisées issues des moyens de prise d'images 22.

Elle est adaptée en particulier pour déterminer la position exacte de l'instrument par rapport à un ensemble de repères optiques comme cela sera décrit dans la suite.

L'unité de traitement d'images 24 est reliée à une unité 26 de traitement d'informations, elle-même reliée à une mémoire 28 adaptée pour mémoriser des images de la zone à traiter préalablement produite par un dispositif d'imagerie médicale non représenté.

En outre, un écran d'affichage 30 est relié à l'unité de traitement d'informations 26 afin d'afficher une image de la tête de travail de l'instrument 14 correctement positionnée dans une image de la zone traitée.

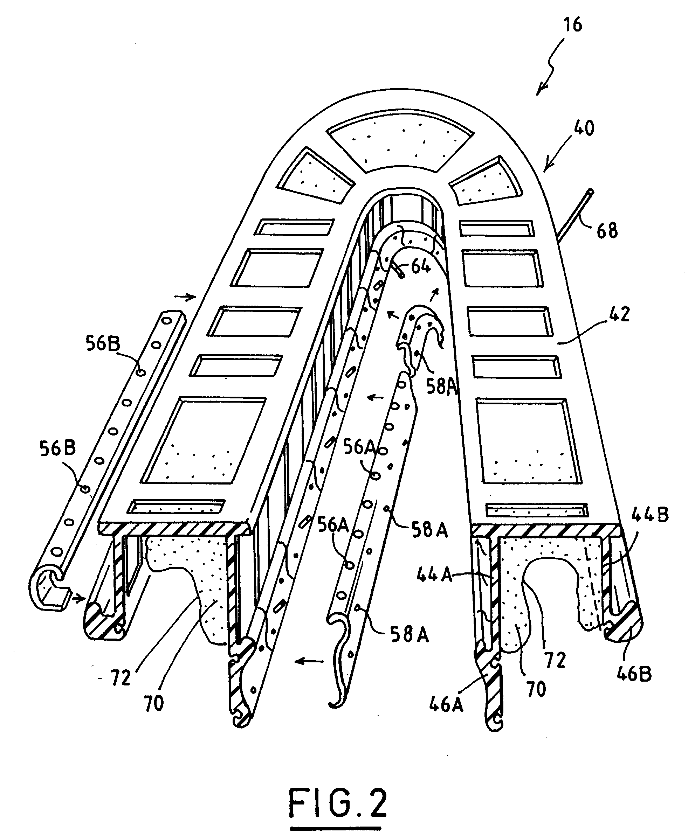

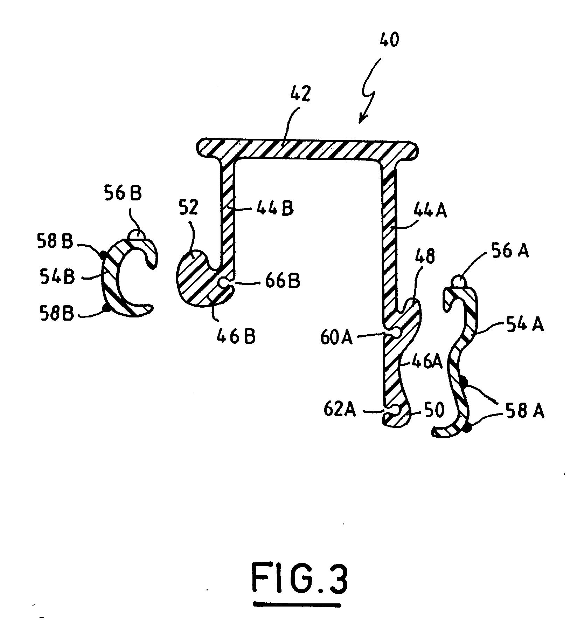

Le support 16 est représenté en détail sur les figures 2 et 3.

Ce support comporte essentiellement une gouttière 40 de section générale en U comportant une face supérieure 42 et deux parois latérales 44A,44B.

La gouttière 40 est repliée en U suivant une forme correspondant à celle de la dentition.

A l'extrémité inférieure des parois latérales 44A, 44B, est prévu extérieurement sur toute la longueur du support 40, un bourrelet d'encliquetage noté respectivement 46A,46B.

Comme représenté sur la figure 3, le bourrelet 46A comporte essentiellement en section, une première saillie 48 orientée vers la face supérieure 42 et une seconde saillie 50 prévue au-dessous de la saillie 48 et orientée généralement à l'opposé de celle-ci.

Le bourrelet 46B comporte en section essentiellement une saillie 52 orientée vers la surface supérieure 42.

Les bourrelets 46A,46B sont destinés à recevoir des flasques 54A,54B.

Ces flasques sont formés par des baguettes élastiques définissant en section sensiblement la forme d'un C et ayant intérieurement un profil correspondant au profil des bourrelets d'encliquetage 46A,46B.

Comme représenté sur la figure 1, les flasques 54A,54B sont adaptés pour s'encliqueter respectivement sur les bourrelets 46A,46B.

Chacun des flasques 54A,54B porte extérieurement sur son aile supérieure, des marques optiques notées 56A,56B. Ces marques optiques sont adaptées pour être visibles sur les images numérisées prises par le moyens optiques 17. Les marques optiques 56A,56B sont réparties régulièrement sur la longueur de chaque flasque comme représenté sur la figure 2.

En outre, chaque flasque 54A,54B comporte dans sa partie inférieure deux rangées parallèles de marqueurs radio-opaques 58A,58B.

Ces marqueurs radio-opaques sont régulièrement répartis sur la longueur des flasques et sont adaptés pour être visibles sur une image numérisée prise par un dispositif d'imagerie médicale adaptée pour produire des images de la zone à traiter.

En outre, la paroi latérale 44A comporte au niveau des saillies 48 et 50 sur sa face intérieure, deux canaux longitudinaux 60A,62A (figure 3) s'étendant sur toute la longueur du support. Ces canaux forment des moyens d'aspiration chirurgicale et sont reliés par un cathéter 64 visible sur la figure 2 à une pompe aspirante.

De même, la paroi latérale 44B comporte sur sa face intérieure, au niveau du bourrelet 46B, un canal 66 (figure 3) s'étendant sur toute la longueur du support. Ce canal forme des moyens d'irrigation et est relié par un cathéter 68 (figure 2) à des moyens de diffusion d'un liquide adapté.

Le support 16 comporte à l'intérieur de la gouttière, des moyens de fixation, portant la référence générale 70, destinés à assurer un maintien du support sur la dentition du patient. Ces moyens de fixation 70 sont par exemple une pâte déformable plastiquement destinée à la prise d'une empreinte dentaire 72.

On conçoit qu'après la prise de l'empreinte 72 sur la dentition du patient, le support 16 peut être retiré, puis repositionné ultérieurement exactement dans sa position initiale en faisant correspondre l'empreinte préalablement enregistrée avec le profil des dents lors de la remise en place du support.

L'installation décrite ici fonctionne de la manière suivante.

Le chirurgien, avant de procéder à l'opération chirurgicale, installe le support 16 sur la dentition du patient. Avant sa mise en place, le support 16 est muni des flasques 54A,54B portant les premier et second ensembles de marques respectivement radio-opaques 58A,58B et optiques 56A,56B.

Le support 16 est pressé convenablement sur les dents du patient, de sorte que ces dernières marquent leur empreinte 72 dans la pâte 70 formant les moyens de fixation.

Alors que le support 16 est immobilisé, un ensemble d'images numérisées de la mâchoire du patient sont enregistrées par un dispositif d'imagerie médicale de tout type connu adapté, notamment un scanner ou un dispositif radiographique à rayons X.

Cet ensemble d'images numérisées fait apparaître en plus de l'image de la mâchoire elle-même, les marques radio-opaques portées par le support 16. Ces images sont mémorisées dans la mémoire 28.

Après l'acquisition de ces images, le support 16 peut être retiré de sorte que le patient peut mener une vie normale jusqu'à l'opération.

Immédiatement avant l'opération, le support 16 est repositionné sur la mâchoire du patient dans la position exacte qu'il avait lors de la prise des images numériques. Ceci est rendu possible par la présence de l'empreinte dentaire permanente 72.

Lors de l'opération chirurgicale, le chirurgien opère avec l'instrument 14 au travers d'ouvertures ménagées dans la partie supérieure 42 du support 16. L'ensemble des marques optiques 56A,56B, disposées sur la partie supérieure des flasques 54A et 54B, sont dans l'angle d'ouverture d'au moins un des moyens d'acquisition optiques 18 prévus sur l'instrument 14.

En effet, chaque prisme disposé sur un côté de l'instrument couvre un angle d'ouverture s'étendant sensiblement sur 180° sur un côté de l'instrument.

Dans ces conditions, on conçoit que les images enregistrées par les moyens de prise d'images 22 font apparaître les marques optiques situées au voisinage de la zone traitée par l'instrument.

L'unité de traitement d'images 24 assure un formatage des images et une identification des éléments d'images représentatifs des marques optiques afin que les images soient exploitables par l'unité de traitement d'informations 26. En particulier, l'unité 24 assure une détermination de la position exacte de l'instrument 14 par rapport aux marques optiques 56A,56B.

Les marques optiques 56A,56B et les marques radio-opaques 58A,58B étant portées par les mêmes flasques, la position relative des marques optiques 56A,56B par rapport aux marques radio-opaques 58A,58B est déterminée avec exactitude.

Ainsi l'unité de traitement d'informations procède à un calcul de la position de l'outil par rapport aux images mémorisées dans la mémoire 28 par un changement de repère représentatif du positionnement relatif des marques optiques par rapport aux marques radio-opaques.

Enfin, l'unité de traitement d'informations 26 assure un affichage sur l'écran 30 de la tête de travail de l'instrument 14 par rapport aux dents.

La position des marques optiques par rapport aux marques radio-opaques étant prédéterminée et parfaitement connue, l'algorithme mis en oeuvre par l'unité de traitement d'informations 26 est relativement simple.

Aussi, le temps de calcul pour engendrer l'image à afficher sur l'écran 30 est relativement réduit, de sorte que l'image peut être régénérée avec une fréquence élevée.

Dans l'exemple décrit ici, les marques radio-opaques et les marques optiques sont portées par un même organe, à savoir les flasques 54A,54B. Toutefois, en variante ces marques sont portées par des organes différents dont la position relative est parfaitement déterminée. En particulier, ces marques sont portées par des organes amovibles fixés successivement dans des positions prédéterminées sur un support commun analogue au support 16.

En outre, le support 16 peut être fixé sur l'organe à traiter par des moyens différents d'une empreinte, et notamment par l'intermédiaire de vis ou d'implants fixes laissés en place dans la zone à traiter.

L'exemple représenté a trait à un dispositif pour le traitement d'une zone de mâchoire en chirurgie dentaire mais on comprend que l'utilisation de moyens équivalents permettra une adaptation à d'autres cas de chirurgie ou microchirurgie.

Suivant un autre mode de mise en oeuvre du procédé de marquage et de repérage, l'ensemble de marques optiques peut être omis.

Ce procédé va maintenant être décrit.

Suivant ce procédé, le médecin ou le chirurgien réalise un ensemble d'images numérisées de la zone à traiter.

Un repérage du positionnement de l'outil au niveau de la zone à traiter est ensuite effectué par repérage optique de la position de l'outil par rapport à la zone à traiter à l'aide des moyens d'acquisition optiques placés en vue directe de la zone et sensibles à la position de l'outil. Les moyens d'acquisition optiques sont avantageusement portés directement par l'outil.

Suivant ce mode de mise en oeuvre du procédé, un procédé de reconnaissance topographique est mis en oeuvre.

Ainsi, afin d'assurer le repérage du positionnement de l'outil, le médecin ou le chirurgien pointe successivement à l'aide de l'outil avant de procéder à l'opération, un ensemble de points de la zone à traiter.

Par ailleurs, il identifie manuellement et visuellement chacun des points sur une ou plusieurs des images numérisées. Cette identification s'effectue par exemple par déplacement d'un index sur une ou plusieurs images numérisées affichées sur un écran.

La position de chacun de ces points de la zone à traiter, ainsi identifiée sur une ou plusieurs images, est mémorisée afin de permettre lors de l'opération chirurgicale un repérage en continu de la position de l'outil.

Suivant une autre variante de mise en oeuvre du procédé, on utilise un procédé de nappage afin d'assurer le repérage de la position de l'outil.

Le procédé de nappage consiste à parcourir avec l'outil la zone à traiter. Lors de ce parcours, on enregistre la variation de la position de l'outil.

A partir d'un algorithme de reconnaissance de profil, on identifie automatiquement la position dudit outil ou instrument par rapport à la zone à traiter grâce à la comparaison de la variation enregistrée de position de l'outil et du profil de la zone à traiter figurant sur la ou les images numérisées.

On comprend qu'avec l'un quelconque des procédés décrit ci-dessus, le positionnement direct des moyens d'acquisition optiques sur ou dans une position fixe par rapport audit outil ou instrument, permet un repérage rapide de la zone à traiter sans qu'il soit nécessaire de mettre en oeuvre lors de l'opération chirurgicale, des algorithmes de calcul complexes et lents.

Par ailleurs, on comprend qu'avec un dispositif tel que décrit ici, dont l'outil est équipé de moyens d'acquisition optiques, il est possible de suivre sur un écran de visualisation, les images prises par les moyens d'acquisition optiques. Ainsi, il est possible de suivre d'une part l'image extérieure de l'outil travaillant dans la zone à traiter et d'autre part, une image tridimensionnelle synthétique de l'outil dans une représentation de la zone à traiter formée à partir des images numérisées.

## Revendications

1. Procédé de marquage et de repérage d'une zone à traiter d'un organisme vivant, et notamment d'une zone non ou mal accessible à la vue du médecin ou chirurgien, comprenant les étapes consistant :
- à fixer sur ladite zone, ou sur une autre zone anatomiquement reliée de façon indéformable à ladite zone, un ensemble initial de marques (58A,58B) formant des points de référence pouvant apparaître sur des images produites par un dispositif d'imagerie médicale,
- à réaliser une ou des images numérisées prises par rapport à l'ensemble de points de référence (58A,58B),
- à fixer sur ladite zone ou une autre zone, un nouvel ensemble de marques optiques (56A,56B), c'est-à-dire exploitable par une acquisition optique, formant des points de référence, le nouvel ensemble de marques se trouvant dans une position parfaitement déterminée par rapport à celle de l'ancien ensemble de marques (58A,58B), et
- à repérer le positionnement d'un outil (14) ou instrument de traitement au niveau de ladite zone à traiter, par repérage optique direct de la position de l'outil ou instrument par rapport audit nouvel ensemble de marques optiques (56A,56B) à l'aide de moyens d'acquisition optiques (18) placés en vue directe dudit nouvel ensemble de marques (56A,56B), et sensibles à la position de l'outil.

2. Procédé selon la revendication 1, caractérisé en ce que lesdits moyens d'acquisition optiques (18) sont directement portés par l'outil ou instrument de traitement, de sorte qu'ils peuvent transmettre, à l'extérieur de l'organisme traité, les données permettant de déterminer le positionnement de l'outil (14) par rapport audit nouvel ensemble de marques (56A,56B).

3. Procédé selon la revendication 1 ou 2, caractérisé en ce qu'il comprend en outre après la réalisation de la ou chaque image numérisée, les étapes consistant :
- à retirer ledit ensemble de points de référence (58A,58B),
- à fixer ultérieurement ledit nouvel ensemble de marques optiques (56A,56B) se trouvant dans une position parfaitement déterminée par rapport à celle de l'ancien ensemble de marques (58A,58B).

4. Procédé selon l'une quelconque des revendications précédentes, caractérisé en ce que le nouvel ensemble de marques (56A,56B) est directement lié géométriquement à la position occupée par l'ancien ensemble de références (58A,58B) fixe ou fixée, qui a servi à l'acquisition de l'image numérisée de la zone de l'organisme.

5. Procédé selon la revendication 4, caractérisé en ce que le nouvel ensemble de marques optiques (56A,56B) est mis en place dans des positions distinctes de celles des marques de l'ensemble initial de marques (58A,58B), mais dont les coordonnées par rapport au système de repère ou référence constitué par l'ancien ensemble de marques (58A,58B), sont connues avec une grande exactitude.

6. Procédé de marquage et de repérage selon l'une quelconque des revendications 1 à 5, d'une zone à traiter d'un organisme vivant, et notamment d'une zone non ou mal accessible à la vue du médecin ou chirurgien, comprenant les étapes consistant :
- à réaliser une ou des images numérisées de ladite zone à traiter, et
- à repérer le positionnement d'un outil ou instrument de traitement au niveau de ladite zone à traiter, par repérage optique direct de position de l'outil ou instrument par rapport à ladite zone à traiter à l'aide de moyens d'acquisition optiques (18) placés en vue directe de ladite zone à traiter, et sensibles à la position de l'outil.

7. Procédé selon la revendication 6, caractérisé en ce que lesdits moyens d'acquisition optiques (18) sont directement portés par l'outil ou instrument de traitement (14), de sorte qu'ils peuvent transmettre, à l'extérieur de l'organisme traité, les données permettant de déterminer le positionnement de l'outil par rapport à ladite zone à traiter.

8. Procédé selon la revendication 6 ou 7, caractérisé en ce qu'il est mis en oeuvre un procédé de reconnaissance topographique, consistant :
- à pointer successivement un ensemble de points de ladite zone à traiter avec ledit outil ou instrument, et
- à identifier manuellement et à mémoriser chacun des points sur une ou plusieurs desdites images numérisées.

9. Procédé selon la revendication 6 ou 7, caractérisé en ce qu'il est mis en oeuvre un procédé de nappage consistant :
- à parcourir avec ledit outil ou instrument ladite zone à traiter,
- à enregistrer lors dudit parcours la variation de position dudit outil ou instrument, et
- à identifier automatiquement la position dudit outil ou instrument par rapport à ladite zone à traiter à partir de ladite variation de position enregistrée dudit outil et du profil de ladite zone à traiter sur la ou les images numérisées.

10. Dispositif de marquage et de repérage destiné à la mise en oeuvre du procédé selon l'une quelconque des revendications 1 à 9, caractérisé en ce qu'il comporte un ensemble de marques optiques (56A,56B) associé à un support (16) muni de moyens de fixation (70) sur ladite zone ou autre zone anatomiquement reliée de façon indéformable, dans une position de l'ensemble de marques optiques (56A,56B) parfaitement déterminée par rapport à la position de l'ensemble initial de marques (58A,58B).

11. Dispositif selon la revendication 10, caractérisé en ce que les moyens de fixation (70) sont agencés pour être fixés exactement à l'emplacement sur lequel ont été initialement fixés les moyens de fixation du support de l'ensemble initial de marques (58A,58B) destiné à l'acquisition par le dispositif d'imagerie médicale, les positions des marques dudit nouvel ensemble de marques (56A,56B) par rapport auxdits moyens de fixation (70) et donc par rapport à l'ensemble initial de marques (58A,58B) étant exactement connues, ce qui permet, sur l'image de l'organe à traiter, d'utiliser le repère de référence formé par le nouvel ensemble de marques (56A,56B) pour calculer et représenter la position de l'outil (14) ou instrument par rapport à l'organe à traiter et, s'il y a lieu, à son image.

12. Dispositif selon l'une quelconque des revendications 10 ou 11 caractérisé en ce que chacun des ensembles de marques est porté par un support respectif de géométrie parfaitement connue et qui vient se fixer sur lesdits moyens de fixation et de positionnement de façon amovible et précise.

13. Dispositif selon l'une quelconque des revendications 10 à 12, caractérisé en ce que les moyens de fixation-en position (70) comportent une empreinte dentaire (72) prise sur la mâchoire et sur laquelle a été fixé, de façon amovible, l'ensemble initial de marques (58A,58B), de sorte qu'en mettant en place l'empreinte (72) exactement dans sa position initiale, le nouvel ensemble de marques (56A,56B) fixé sur ladite empreinte (72) occupera une position parfaitement définie, c'est-à-dire connue par rapport à celle de l'ensemble initial de marques (58A,58B) qui avait été fixé sur l'empreinte (72).

14. Dispositif selon l'une quelconque des revendications 10 à 12, caractérisé en ce que les moyens de fixation comportent des vis ou implants fixes laissées en place dans la zone à traiter.

15. Dispositif selon l'une quelconque des revendications 10 à 14, caractérisé en ce que les moyens d'acquisition optique (18) susceptibles de déterminer le position de l'outil (14) ou instrument par rapport à l'ensemble de marques optiques sont directement portés par l'outil ou l'instrument (14).

16. Outil ou instrument de traitement pour la mise en oeuvre du procédé selon l'une quelconque des revendications 1 à 9 portant des moyens optiques permettant de repérer la position de l'instrument (14) par rapport aux marques optiques du nouvel ensemble de marques (56A, 56B).

17. Outil selon la revendication 16, caractérisé en ce que les moyens optiques comprennent des fibres optiques (20) se terminant par une ou des optiques appropriées (18) disposées sur ledit outil ou instrument (14), ces fibres (20) émergeant à distance de l'organisme, loin de l'instrument (14), pour être raccordées à des moyens (22, 24) permettant, à partir des images optiques des marques transmises par les fibres, de déterminer la position exacte de l'outil ou instrument (14) par rapport aux repères formés par ledit nouvel ensemble de marques (56A, 56B).

18. Outil selon la revendication 17, caractérisé en ce qu'il comporte une ou plusieurs sources lumineuses susceptibles d'éclairer lesdites marques optiques.

19. Outil selon la revendication 18, caractérisé en ce que les sources lumineuses sont constituées par les extrémités, pourvues d'optiques diffusantes convenables, de fibres optiques aboutissant à l'instrument et reliées, à leurs autres extrémités, à une source lumineuse.

20. Ensemble constitué par un dispositif de marquage et de repérage selon l'une quelconque des revendications 10 à 15 et un outil ou instrument de traitement (14) selon l'une quelconque des revendications 16 à 19, portant lesdits moyens d'acquisition optiques (18).
